# EUROPEAN PATENT APPLICATION

(11) **EP 0 608 948 A2**
(43) Date of publication of application: **03.08.1994**
(21) Application number: 94200163.7
(22) Date of filing: 24.01.1994
(51) Int. Cl.: A01K 11/00, A01K 29/00, A61B 5/11

(54) **Activity monitors**

(30) Priority: 26.01.1993 GB 9301502
(71) Applicant: MINISTER OF AGRICULTURE, FISHERIES AND FOOD IN HER BRITANNIC MAJESTY'S GOV. OF THE U.K. OF GREAT BRITAIN AND NORTHERN IRELAND, London SW1A 2HH (GB)
(72) Inventor: Pearson, Colin Charles, Tilehurst, Reading, Berkshire RG3 5AU (GB); Barnes, Robin Noel, Murrow, Wisbeach,Cambridgeshire PE13 5SQ (GB)
(74) Representative: Lockwood, Peter Brian

(57) **Abstract**

According to one aspect of the present invention a method of monitoring activity of at least one animate being includes the steps of monitoring the being with a detector (10) such as a dual element pyro-electric sensor capable of detecting movement and of recording on a logging device (12), at predetermined intervals, the presence or absence of movement. The logging device (12) preferably contains means for averaging the records over a predetermined time scale and of storing the average in a memory. By repeating this process over a period of time a record of the being's activity can be obtained.

## Description

The present invention is concerned with apparatus and methods for studying activity, particularly the activity of animals.

A great deal of research is being conducted into improving the living conditions of animals concerned with the food industry. One criterion of the welfare of such animals is their level of activity. Current methods of measuring the activity involve, either the use of a trained observer to record activity onto paper or into a manually operated logging device, or the use of video or photographic recording of the animals with subsequent analysis of the records. Both these methods are time consuming and expensive.

There is therefore an need for an improved method of carrying out this type of research.

According to one aspect of the present invention a method of monitoring activity of at least one animate being includes the steps of monitoring the being with a detector capable of detecting movement and of recording on a logging device, at predetermined intervals, the presence or absence of movement.

The logging device preferably contains means for averaging the records over a predetermined time scale and of storing the average in a memory. By repeating this process over a period of time a record of the being's activity can be obtained.

The detector might be, for example, a dual element pyro-electric sensor, with a Fresnel lens, which detects changes in its infra-red field of view. Such detectors are commonly used in burglar alarms.

The predetermined intervals and the predetermined time scale might be, for example, three seconds and five minutes respectively.

In circumstances where it is required to monitor the activities of one of a number of adjacent beings that one of the beings might be adapted to provide an enhanced signal to which the detector responds. For example, where the detector is an infra-red detector the being might be fitted with an infra-red emitter.

According to another aspect of the invention an activity monitor includes a detector adapted to detect motion, and a logging device connected to the detector and adapted to log, at predetermined intervals, the presence or absence of motion.

In use the activity monitor will be connected to a power supply. The logging device will preferably be adapted to average the readings over a predetermined time scale and to store the average, to record the average, for example on a chart, or both.

A preferred form of monitor has an infra red detector, which might be a passive sensor such as, for example, a dual element pyro-electric sensor with a Fresnel lens of the type commonly used in burglar alarms.

The functions of the logger might conveniently be controlled by a micro-computer.

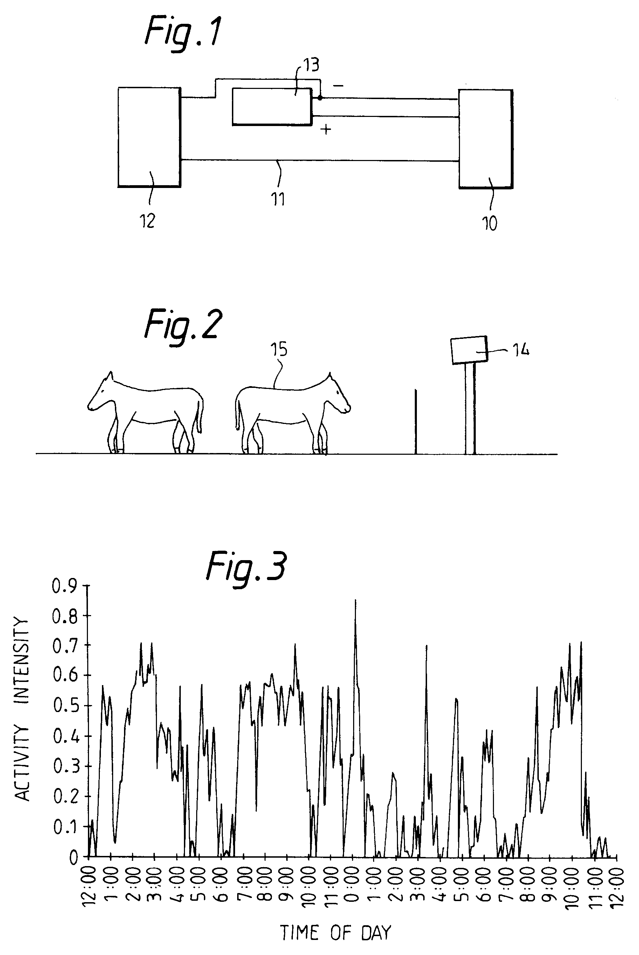

One embodiment of the invention will now be described, by way of example only, with reference to the accompanying diagrammatic drawings, of which: Figure 1 shows a schematic view of a monitoring device according to the invention,
Figure 2 shows the device adapted to take measurements, and
Figure 3 shows a typical activity record taken by the monitor.

A monitoring device (Figure 1) includes a dual element pyro-electric infrared sensor 10, of the type having a Fresnel lens and as used in burglar alarms. The sensor 10 has an output 11 to an electronic logger 12 which contains a programmed micro-computer (not shown).

In use, the sensor 10 and logger 12 are connected to a power supply in the form of a battery 13 and enclosed in a box 14 (Figure 2). The box 14 is positioned relative to an animal 15, whose activity it is desired to monitor, such that the sensor 10 is overlooking the animal 15.

At predetermined intervals, for example every three seconds, the logger 12 records whether or not the sensor is indicating activity of the animal 15. The records are averaged over a period, for example five minutes, and plotted against a suitable base such as the time of day (Figure 3).

It will be realised that many other types of sensor, infra red or other, may be used with the invention- such as, for example, radar apparatus or video cameras. Also power supplies other than batteries may be used, and the logger 12 may be positioned remotely from the sensor 10, perhaps with separate power supplies for sensor and logger. The link 11 need not be a direct link but might, for example, be a radio link, facilitating movement of the sensor 10 between various measuring stations, with the logger positioned at some central point.

When the monitor is used as described above for monitoring the activities of a single animal, and this animal is one of a number, the animal might have attached thereto means providing an enhanced signal to which the sensor responds. Alternatively a monitor might be adapted to sense movement of any one of a number of animals, the average activity of a single animal being obtained in the calculation process.

It will be realised that sensors are available whose operative ranges and sensitivities can be adjusted as required.

It will also be realised that whilst the monitor has been described above as being used for the monitoring of animal activity, monitors according to the invention, and the method of the invention, can be used for other purposes; for example in the study of human crowds, on fish farms, and for bee hives.

## Claims

1. A method of monitoring activity of at least one animate being including the steps of monitoring the being with a detector capable of detecting movement and of recording on a logging device, at predetermined intervals, the presence or absence of movement.

2. A method as claimed in Claim 1 wherein the logging device contains means for averaging the records over a predetermined time scale and of storing the average in a memory.

3. A method as claimed in Claim 2 wherein a record of a series of average readings is obtained.

4. A method as claimed in Claim 2 or in Claim 3 wherein the predetermined time scale is five minutes.

5. A method as claimed in any one of Claims 1 to 4 wherein the predetermined intervals are three seconds.

6. A method as claimed in any one of Claims 1 to 5 wherein the being is adapted to provide an enhanced signal to which the detector responds.

7. An activity monitor including a detector adapted to detect motion, and a logging device connected to the detector and adapted to log, at predetermined intervals, the presence or absence of motion.

8. A monitor as claimed in Claim 7 including a power supply.

9. A monitor as claimed in Claim 7 or in Claim 8 wherein the logging device is adapted to average the readings over a predetermined time scale.

10. A monitor as claimed in Claim 9 wherein the logging device is programmed to provide a record of average readings over a period of time.

11. A monitor as claimed in any one of Claims 7 to 10 wherein the detector is an infra red detector.

12. A monitor as claimed in Claim 11 wherein the infra red detector is passive sensor.

13. A monitor as claimed in Claim 12 wherein the sensor is a dual element pyro-electric sensor.

14. A monitor as claimed in Claim 13 wherein the sensor has a Fresnel lens.

15. A method of monitoring activity substantially as herein described with reference to figures 1 to 3 of the accompanying drawings.

16. A method of monitoring activity substantially as herein described.

17. An activity monitor substantially as herein described with reference to Figures 1 to 3 of the accompanying drawings.

18. An activity monitor substantially as herein described.
